Europäisches Patentamt

European Patent Office

Office européen des brevets

(19)

(11) Numéro de publication: **0 244 285 B1**

(12) **FASCICULE DE BREVET EUROPEEN**

(45) Date de publication du fascicule du brevet:
29.08.90

(51) Int. Cl.⁵: **A61K 9/72, A61K 33/00**

(21) Numéro de dépôt: **87400797.4**

(22) Date de dépôt: **09.04.87**

(54) **Produit de radiosensibilisation des tissus biologiques en radiothérapie.**

(30) Priorité: **14.04.86 FR 8605277**

(43) Date de publication de la demande:
**04.11.87 Bulletin 87/45**

(45) Mention de la délivrance du brevet:
**29.08.90 Bulletin 90/35**

(84) Etats contractants désignés:
**AT BE CH DE ES FR GB GR IT LI LU NL SE**

(56) Documents cités:
**GB-A- 1 396 772**

(73) Titulaire: **L'AIR LIQUIDE, SOCIETE ANONYME POUR L'ETUDE ET L'EXPLOITATION DES PROCEDES GEORGES CLAUDE, 75, Quai d'Orsay, F-75321 Paris Cédex 07(FR)**

(72) Inventeur: **Mondain-Monval, Gérard, 51, boulevard Soult, F-75012 Paris(FR)**

(74) Mandataire: **Sadones Laurent, Renée et al, L'AIR LIQUIDE 75, quai d'Orsay, F-75321 Paris Cédex 07(FR)**

ACTORUM AG

## Description

La présente invention concerne un produit de radiosensibilisation des tissus biologiques en radiothérapie.

On sait que les cellules bien "oxygénées" sont plus sensibles aux radiations et qu'au contraire l'anoxie diminue la radiosensibilité. Or les cellules tumorales sont souvent peu "oxygénées" donc peu sensibles aux radiations thérapeutiques.

L'oxygénothérapie hyperbare permet d'augmenter l'oxygénation des cellules tumorales et par conséquent de les rendre plus vulnérables aux rayonnements, et améliorer ainsi l'efficacité d'une irradiation thérapeutique. Il a donc été développé une thérapeutique combinant radiothérapie et oxygénothérapie hyperbare, selon laquelle le patient est placé sous oxygène dans un caisson hyperbare monoplace transparent aux radiations et placé sous les générateur de rayonnement (X ou γ - cobalt).

Mais le développement de cette thérapeutique est ralenti par le coût et la lourdeur de mise en œuvre et de l'équipement : caisson hyperbare, protocole et surveillance à distance du patient placé dans le caisson et sous irradiation, processus de compression et décompression, précaution de sécurité en raison de l'emploi d'oxygène pur dans le caisson, etc...

Afin d'augmenter l'oxygénation des cellules sans employer l'oxygénothérapie hyperbare, on a proposé l'oxygénothérapie "normobare". Cette thérapeutique consistant en l'inhalation par le patient à la pression ambiante non plus de l'air mais de l'air suroxygéné voire de l'oxygène pur, représente déjà une première amélioration, mais ne permet d'augmenter que légèrement la quantité d'oxygène disponible dans le sang et les tissus.

Aussi, il a été recherché un moyen efficace de radiosensibiliser les cellules à traiter par radiothérapie.

Un mélange gazeux contenant au moins de l'oxygène et du protoxyde d'azote permet d'obtenir cet effet de manière très satisfaisante.

Le protoxyde d'azote est déjà connu en tant que produit utilisable notamment dans le domaine thérapeutique, ainsi le brevet britannique 1 396 722 fait référence aux propriétés analgésiques qu'il manifeste et mentionne l'action anesthésique des mélanges de protoxyde d'azote, d'anhydride carbonique et d'oxygène.

Il a été découvert une nouvelle fonction d'un mélange contenant du protoxyde d'azote, et correspondant à un rôle de radiosensibilisateur sélectif ou différential entre les cellules saines et tumorales, cellules nerveuses lipidiques, et membranes cellulaires protéiniques, n'ayant aucun lien avec les fonctions analgésiques et anesthésiques connues.

Le produit de radiosensibilisation est inhalé par le patient soumis à la radiothérapie. Cet emploi nouveau du protoxyde d'azote ne pose aucun problème particulier au corps médical. En effet, ce dernier utilise couramment le protoxyde d'azote en anesthésie et analgésie, et en a par conséquent une parfaite connaissance sur les plans physiologiques, sécurité, matériel et technique d'emploi.

L'intérêt d'un produit de radiosensibilisation des tissus constitué par un mélange gazeux contenant au moins 50% en volume de protoxyde d'azote, de préférence 50 à 80% en volume, et au moins 20% en volume d'oxygène a été apprécié.

Le produit de radiosensibilisation des tissus biologiques peut se présenter sous forme d'un mélange binaire constitué par de l'oxygène et du protoxyde d'azote, préparé soit à l'avance en prémélange, soit extemporanément au moyen d'un mélangeur protoxyde d'azote-oxygène, par un mélangeur dit de sécurité qui délivre un mélange gazeux dont la teneur en oxygène est au minimum de 20% en volume.

Le produit de radiosensibilisation des tissus biologiques peut aussi se présenter sous forme d'un mélange ternaire constitué par de l'oxygène, du protoxyde d'azote et le complément à 100 en volume par un gaz inerte, choisi parmi l'azote, l'argon, le krypton, le xénon et l'hélium.

Les deux types de mélanges binaire et ternaire peuvent être préconditionnés sous des pressions compatibles avec le maintien des mélanges sous forme gazeuse.

L'administration du mélange gazeux contenant du protoxyde d'azote est réalisée par inhalation à l'aide de matériels simples et peu coûteux, par exemple du type masque et sonde d'intubation.

On fait respirer le produit de radiosensibilisation au patient préalablement au traitement d'irradiation, pendant une durée suffisante de 15 à 30 minutes pour atteindre la saturation des tissus biologiques.

Les différents tissus se saturent ou se désaturent en protoxyde d'azote plus ou moins rapidement. Schématiquement on peut classer les tissus en trois catégories principales selon leur constante de temps saturation - désaturation. Les tissus de première catégorie ont une constante de temps de l'ordre de 5 minutes, pour la deuxième catégorie la constante de temps est de l'ordre de 15 minutes et pour la troisième catégorie cette constante atteint l'ordre d'une heure.

Ce paramètre "temps" peut être mis à profit de la manière suivante: par exemple après une heure d'inhalation d'un mélange gazeux riche en protoxyde d'azote, c'est-à-dire contenant plus de 50% de ce gaz, on peut considérer que l'organisme est saturé. Si l'on interrompt l'inhalation de protoxyde d'azote, au bout de 5 minutes, on peut estimer que la première catégorie de tissus aura éliminé le protoxyde d'azote et par conséquent si l'on procède alors à une irradiation, ces tissus ne contenant plus de protoxyde d'azote seront moins vulnérables, alors que les tissus des 2ème et 3ème catégorie contenant encore du protoxyde d'azote seront radiosensibilisés et détruits par le rayonnement. Cet exemple met en évidence

2

l'effet différentiel ou sélectif que l'emploi du protoxyde d'azote permet d'obtenir dans cette application, en particulier dans les traitements des cancers par radiothérapie.

En outre, le facteur de différenciation résulte aussi de la solubilité du protoxyde d'azote selon le type de tissus cellules saines ou tumorales, lipidiques ou protéiniques.

Une irradiation débutée après 30 minutes d'arrêt de l'inhalation du protoxyde d'azote, est efficace sélectivement sur les tissus de la troisième catégorie.

L'inhalation du produit de radiosensibilisation peut être poursuivie au cours de l'irradiation quand on veut atteindre une forte concentration en protoxyde d'azote, soit dans le cas d'irradiation intense peropératoire, et soit d'une manière plus générale dans les cas où l'on peut délimiter la zone d'irradiation.

L'effet radiosensibilisant de l'inhalation d'un mélange de protoxyde d'azote et d'oxygène a été mis en évidence sur des lots de souris saines. On a contrôlé l'effet de l'irradiation sur l'animal entier, inhalant un mélange non hypoxiant de protoxyde d'azote - oxygène ($N_2O$–$O_2$). L'irradiation a été délivrée par une source de cobalt 60. Les souris sont mises dans une boite transparente aux rayonnements compartimentée en 30 logettes percées chacune d'orifices de ventilation sur la face postérieure. Le dispositif est placé à 1 mètre de la source dans le champ d'irradiation. Pour assurer l'inhalation du mélange à tester la boite est hermétiquement enfermée dans un sac plastique mince et ventilé à 5 lit/min par le mélange gazeux qui est rejeté dans une cheminée d'évacuation. Dans toutes les expériences, l'exposition des souris à un mélange donné a toujours débuté 30 minutes avant le début de l'irradiation. Le résultat a été jugé par la mortalité pendant les 30 premiers jours.

Il a été effectué quatre séries d'expériences dont les résultats sont consignés dans le tableau suivant.

Sur ce tableau, la comparaison entre les expériences isodose dans l'air et dans le mélange $N_2O/O_2$ montre que, dans tous les cas, l'inhalation de protoxyde d'azote entraine une radiosensibilisation.

Plus précisément, la première série d'expérience donne les doses léthales à 50 p. cent sur 30 jours, DL $_{50} \pm 20$, suivantes

8,10 $\pm$ 0,15 Gy dans l'air

7,55 $\pm$ 0,15 Gy dans le mélange $N_2O/O_2$

La différence est très significative $p < 0,001$.

La deuxième série a donné de même

8,60 $\pm$ 0,15 Gy dans l'air

7,60 $\pm$ 0,15 Gy dans le mélange $N_2O/O_2$, la différence est également très significative $p < 0,001$.

Les deux dernières séries d'expériences ont servi à confirmer les premiers résultats et l'on constate que ces résultats sont très comparables.

Ainsi, de façon très significative ($p < 0,001$) l'inhalation d'un mélange à 76% $N_2O$/24% $O_2$ rend la toxicité de l'irradiation, dans les conditions des expériences 10 pour cent plus forte, environ, en présence de protoxyde d'azote.

Cet effet radio-sensibilisant du protoxyde d'azote peut être utilisé pour accroitre l'efficacité de la radiothérapie.

TABLEAU

| Série | Nature du mélange | Irradiation | | | | | Morts dans les 30j | | | Expérience N° |
|---|---|---|---|---|---|---|---|---|---|---|
| | | | Nb de souris | Dose Gy | Inten-sité Gy/min | Durée min | Nb | délai moyen jours | | |
| I | Air (témoins) | | 30 a | 8,5 | 0,65 | 13,08 | 23 | 12,1 | | 1 |
| | | | 30 a | 8,0 | 0,65 | 12,30 | 15 | 15,3 | | 2 |
| | | | 30 a | 7,5 | 0,65 | 11,29 | 5 | 13,3 | | 3 |
| | $N_2O$ 76% $O_2$ 24% | | 30 a | 8,0 | 0,65 | 12,30 | 17 | 13,7 | | 4 |
| | | | 30 a | 8,5 | 0,65 | 13,08 | 28 | 11,5 | | 5 |
| | $N_2O$ 76% $O_2$ 24% | | 30 a | 8,0 | 0,65 | 12,30 | 23 | 13,0 | | 6 |
| | | | 30 a | 7,5 | 0,65 | 11,29 | 14 | 13,0 | | 7 |
| II | Air (témoins) | | 30 b | 9,0 | 0,63 | 14,29 | 20 | 15,2 | | 8 |
| | | | 30 b | 8,5 | 0,63 | 13,49 | 9 | 15,4 | | 9 |
| | | | 30 b | 8,0 | 0,63 | 12,70 | 6 | 14,5 | | 10 |
| | | | 30 b | 7,5 | 0,63 | 11,90 | 3 | 22,3 | | 11 |
| | $N_2O$ 76% $O_2$ 24% | | 30 b | 8,5 | 0,63 | 13,49 | 26 | 14,0 | | 12 |
| | | | 30 b | 8,0 | 0,63 | 12,70 | 23 | 14,1 | | 13 |
| | | | 30 b | 7,5 | 0,63 | 11,90 | 13 | 15,3 | | 14 |
| III | Air (témoins) | | 30 b | 7,5 | 0,61 | 12,30 | 7 | 14,4 | | 15 |
| | $N_2O$ 76% $O_2$ 24% | | 30 b | 7,5 | 0,61 | 12,30 | 12 | 13,75 | | 16 |
| IV | Air (témoins) | | 30 b | 8,5 | 0,59 | 14,41 | 5 | 14,0 | | 17 |
| | | | 30 b | 8,0 | 0,59 | 13,56 | 4 | 12,1 | | 18 |
| | $N_2O$ 76% $O_2$ 24% | | 30 b | 8,5 | 0,59 | 14,41 | 27 | 13,8 | | 19 |
| | | | 30 b | 8,0 | 0,59 | 13,56 | 17 | 12,5 | | 20 |

a : Souris albinos femelles, Souche 17 de l'Institut Curie, âge : 4 mois, poids : 22 à 24 g.

b : Souris mâles, Souche CDI production V.A.F. de Charles River, âge : 3 mois, poids : 25 à 30 g.

Gy: Gray unité d'irradiation.

# EP 0 244 285 B1

## Revendications

1. Emploi du protoxyde d'azote dans la préparation d'un produit de radiosensibilisation des tissus biologiques en radiothérapie du cancer, caractérisé en ce que l'on mélange en phase gazeuse de l'oxygène à du protoxyde d'azote en vue de la préparation d'un mélange non-hypoxiant, ledit mélange gazeux de radiosensibilisation non hypoxiant contenant de 50 à 80% en volume de protoxyde d'azote, et au moins 20% en volume d'oxygène.

2. Emploi du protoxyde d'azote dans la préparation d'un produit de radiosensibilisation selon la revendication 1, caractérisé en ce que le mélange gazeux est sous forme ternaire constitué par de l'oxygène et du protoxyde et contient en outre un gaz inerte en complément à 100.

3. Emploi du protoxyde d'azote dans la préparation d'un produit de radiosensibilisation selon la revendication 2, caractérisé en ce que le gaz inerte est choisi parmi l'azote, l'argon, le krypton, le xénon et l'hélium.

4. Emploi du protoxyde d'azote dans la préparation d'un produit de radiosensibilisation selon une quelconque des revendications 1 à 3, caractérisé en ce que le produit est préconditionné sous des pressions compatibles avec le maintien du mélange sous forme gazeuse.

5. Emploi du protoxyde d'azote dans la préparation d'un produit de radiosensibilisation selon une quelconque des revendications 1 à 4, caractérisé en ce que le mélange gazeux est préparé ex-temporanément au moyen d'un mélangeur délivrant un mélange gazeux dont la teneur en oxygène est au minimum de 20% en volume.

## Patentansprüche

1. Verwendung von Stickstoffprotoxid bei der Herstellung eines Produktes für die Radiosensibilisierung biologischer Gewebe in der Radiotherapie von Krebs, dadurch gekennzeichnet, daß man in Gasphase Sauerstoff mit Stickstoffprotoxid unter Herstellung eines nicht-hypoxischen Gemisches vermischt, wobei dieses nicht-hypoxischen gasförmige Gemisch für die Radiosensibilisierung 50 bis 80 Vol.-% Stickstoffprotoxid und wenigstens 20 Vol.-% Sauerstoff enthält.

2. Verwendung von Stickstoffprotoxid bei der Herstellung eines Produktes für die Radiosensibilisierung nach Anspruch 1, dadurch gekennzeichnet, daß das gasförmige Gemisch in ternärer Form vorliegt und außer dem Sauerstoff und Protoxid ein Inertgas in Ergänzung auf 100 enthält.

3. Verwendung von Stickstoffprotoxid bei der Herstellung eines Produktes für die Radiosensibilisierung nach Anspruch 2, dadurch gekennzeichnet, daß das Inertgas unter Stickstoff, Argon, Krypton, Xenon und Helium ausgewählt ist.

4. Verwendung von Stickstoffprotoxid bei der Herstellung eines Produktes für die Radiosensibilisierung nach einem der Ansprüche 1 bis 3, dadurch gekennzeichnet, daß das Produkt unter Drücken, die mit der Aufrechterhaltung des Gemisches in Gasform verträglich sind, vorkonditioniert wird.

5. Verwendung von Stickstoffprotoxid bei der Herstellung eines Produktes für die Radiosensibilisierung nach einem der Ansprüche 1 bis dadurch gekennzeichnet, daß das gasförmige Gemisch auf der Stelle mit Hilfe eines Mischers bereitet wird, der ein Gasgemisch liefert, dessen Sauerstoffgehalt mindestens 20 Vol.-% beträgt.

## Claims

1. Use of nitrous oxide in the preparation of a product for radiosensitizing biological tissues in cancer radiotherapy, characterized in that oxygen and nitrous oxide are mixed in the gaseous phase in order to prepare a nonhypoxic mixture, the said non-hypoxic gaseous radiosensitizing mixture containing from 50 to 80% by volume of nitrous oxide and at least 20% by volume of oxygen.

2. Use of nitrous oxide in the preparation of a radiosensitizing product according to claim 1, characterized in that the gaseous mixture is in ternary form constituted by oxygen and nitrous oxide and further contains an inert gas making it up to 100.

3. Use of nitrous oxide in the preparation of a radiosensitizing product according to claim 2, characterized in that the inert gas is selected from nitrogen, argon, krypton, xenon and helium.

4. Use of nitrous oxide in the preparation of a radiosensitizing product according to any one of claims 1 to 3, characterized in that the product is preconditioned under pressures compatible with the maintenance of the mixture in gaseous form.

5. Use of nitrous oxide in the preparation of a radiosensitizing product according to any one of claims 1 to 4, characterized in that the gaseous mixture is prepared extemporaneously by means of a mixer delivering a gaseous mixture the oxygen content of which is at least 20% by volume.